# EUROPEAN PATENT SPECIFICATION

(11) **EP 1 622 552 B1**
(45) Date of publication and mention of the grant of the patent: **26.03.2008**
(21) Application number: 04732576.6
(22) Date of filing: 13.05.2004
(51) Int. Cl.: A61F 5/44, A61M 1/00

(54) **A BAG FOR COLLECTING LIQUID**
BEUTEL ZUR FLÜSSIGKEITSAUFNAHME
SACHET DE COLLECTE DE FLUIDE

(30) Priority: 14.05.2003 DK 200300732
(43) Date of publication of application: 08.02.2006
(73) Proprietor: Unomedical A/S, 3460 Birkerød (DK)
(72) Inventor: STISEN, Borge, 3230 Græsted (DK)
(74) Representative: Sundien, Thomas
(86) International application number: PCT/DK2004/000342
(87) International publication number: WO 2004/100842

(56) References cited:
- WO-A-93/17643
- US-A- 4 270 533
- US-A- 4 421 509
- US-A- 4 449 969
- US-A- 4 581 763

## Description

The present invention relates to a bag for holding a liquid, in particular urine, said bag having an inlet opening for the liquid and comprising a front formed of a flexible material and a rear formed of a flexible material and being connected to the front along an annularly extending connecting area for forming the sides of a chamber for holding said liquid, and said bag having at the top holder devices, by means of which the bag can be carried in a suspended position, the front and rear of the bag being configured for assuming a bulging shape, when liquid has collected therein.

Bags of this kind lend themselves for use for many different applications, including eg for holding urine supplied to the bag from eg a bedridden patient. Bags for the latter purpose are used in hospitals, in particular in intensive care units, and are supplied with urine from a measurement container that registers the amount of urine discharged by the patient. Typically the bag is mounted on the side of the bed, eg on the bed frame. Since modem beds can be adjusted in height, the bottom of the bed and thus a discharge valve, if any, at the bottom of the bag could typically be situated in varying heights above the floor in response to the setting of the bed.

Prior to filling, the prior art bags are relatively flat and exhibit a plane front connected to a plane rear. In case of urine collecting bags, holder means in the form of openings or pockets are provided at the top that enable attachment of the bag to the bottom of said measurement container by hinging. At the top, the bag has an inlet opening that is connected to the discharge spout from the measurement container. Typically, a one-way valve is provided within the bag at the inlet opening, thereby preventing urine from returning to the measurement container if the bag is laid down.

It is a common feature of the prior art bags that, when the interior chamber between the front and the rear is replete with liquid, it assumes, seen from the side, a bulging shape which is tapering, upwardly as well as downwardly. In order to achieve sufficient liquid-holding capacity the bags must be manufactured with a comparatively large height or alternatively a comparatively wide width. The terms "height" and "width" as used herein are intended to designate the dimensions of the bag in the empty and non-bulging state.

When bags are used that have a wide width, however, there is an ensuing risk of the bag forming, during filling, pockets near the vertical side edges of the bag, and it may be difficult in a subsequent procedure for emptying the bag to force the liquid out of these pockets. This is a problem, in particular when bags are concerned that are provided with a discharge valve centrally at the bottom of the bag, as is the case with bags for collecting and holding urine from a patient. Conversely, when bags with large heights are used, space problems may arise, which may also be a problem when urine bags are concerned, in which case there has to be room for suitable discharge equipment underneath the discharge valve, and where the bag is suspended from a bed set to low height.

Thus a bag is needed that may, for a given capacity, have relatively small height and width dimensions. This is accomplished as featured in the characterising part of claim 1 in that, interiorly of the chamber between the flexible front and the flexible rear, means are arranged for transferring at least a part of the weight of the liquid contained in the chamber to the holder device; and that the front and/or rear exhibit(s), in the empty state of the bag, at least one fold that extend(s) transversally of the bag.

US 4,581,763 teaches a urine collecting bag with pieces of foil arranged interiorly of the bag chamber. The construction of the bag does not enable a reduction in the height without consequences to the liquid capacity of the bag.

The bag according to the invention can be manufactured in a conventional manner by joining two foil sheets by welding and thus forming a closed chamber. According to one embodiment one or more further foil sheets may be attached by welding within the bag, said sheets constituting said means, between the holder devices that may be configured as suspension openings at the top of the bag and the lower edge of the bag. Said further foil sheets secure the lower edge of the bag in relation to the suspension openings and partially take up the weight of the urine. Hereby the front and rear are relieved, thereby enabling them to bulge whereby the bag becomes bulkier compared to the rather tapering shape that characterises the prior art bags. The further foil sheets may also be arranged to create a sloping drop in a direction towards the discharge valve of the bag.

The invention also relates to a method of manufacturing a bag, wherein the constituent foils can be assembled in plane shape, there being, for instance in a subsequent heat treatment - eg in connection with sterilisation, provoked a back-folding of the innermost foil its original, short state.

The invention will be explained in further detail in the following with reference to the drawing, wherein
Figure 1 shows a urine collecting bag according to the invention during its manufacture;
Figure 2 shows the urine collecting bag shown in Figure 2 at a later stage in its manufacture;
Figure 3 shows the finished urine collecting bag shown in Figures 1 and 2 suspended underneath a measurement container and containing a liquid;
Figure 4 shows a possible construction of the bag, seen from the side in a cross-sectional view and in empty state;
Figures 5a and 5b show a further possible construction of the bag, seen from the side in cross-sectional view in the empty state and in the unfolded state of the foil sheet, respectively; and
Figure 6 shows a particular embodiment, wherein foils forming the bag front and rear are shaped tree-dimensionally.

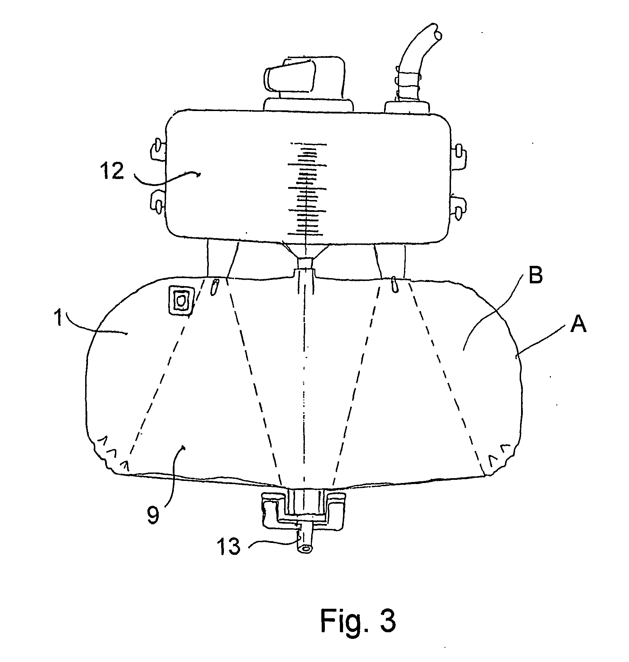

In Figure 3, the letter A indicates a bag according to the invention mounted on the bottom side of a measurement container 12 for measuring the discharge of urine from a patient. In the measurement container, the amount of urine can be determined manually by reading of the liquid level on a calibration, or electronically. In both cases the urine in the measurement container is allowed to enter the interior chamber B in the bag A at intervals, said bag being suspended underneath the measurement container 12. At the bottom of the bag A, a valve 13 is provided that can be operated to empty the collecting bag. Thus, typically the measurement container 12 is emptied once an hour, and the bag a few times every 24 hours. The volume of such bag is typically 2000 ml. The bags are supplied to hospitals either mounted on the measurement container or as an independent product.

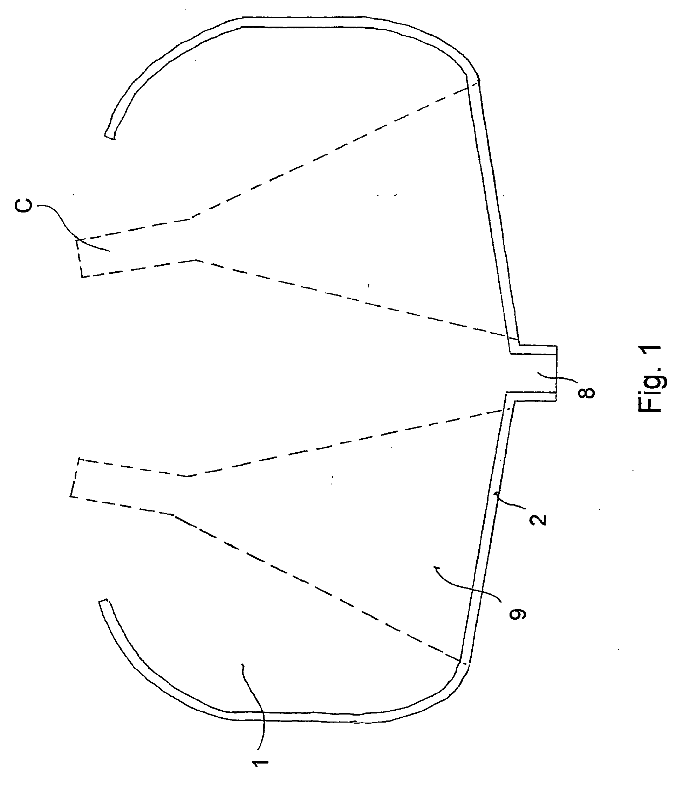

Figure 1 shows an early stage in the manufacture of a bag consisting of two foil sheets 1 that form the front and the rear of the bag, respectively. The foil sheets are joined by means of a welding seam 2 along the sides and lower edge of the chamber B. Along the lowermost edge and at the welded joint of the foil sheets, two additional foil sheets 9 are also provided that are both configured with a narrow upper attachment portion C. In the lower welded joint, an opening 8 is configured in a conventional manner for the discharge valve. The opening 8 may be reinforced by means of a tubular element, in which the discharge valve is mounted.

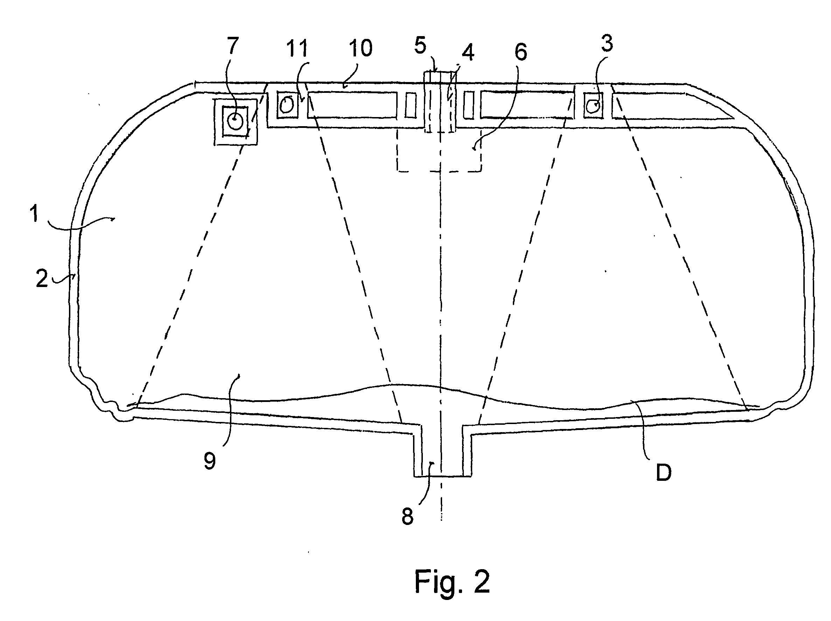

Figure 2 shows a later stage in the manufacture. It will appear how the foil sheets 9 are pulled upwards, the attachment portion C being joined by welding to the foil sheets 1 along a portion of the upper connecting area 10 of the bag and optionally reinforced by the welding lines 11. When the foil sheets 9 are welded both along the welding seam 2 and the upper welding seam 10, the foil sheets 1 will be folded and form a fold D that extends transversally of the bag A. This is due to the fact that the distance between the welding seam 10 and the opposite welding most proximate the lower edge of the bag A - and hence the extent of the foil sheets 9 - is smaller than the extent of the foil sheets 1 that form the front and rear of the bag A.

At the top of the bag, outside the chamber B, suspension openings 3 are provided that constitute holder devices for the bag, by means of which the bag can be carried in a suspended position. In the upper seam 10 there is, in a conventional manner, formed an inlet opening 4 for connecting the chamber B to a measurement container. The opening 4 can be reinforced by a tubular element 5. At the opening 4 within the chamber B, separate foil elements 6 can be provided that form a one-way valve, whereby liquid is prevented from returning to the measurement container 12 if the bag A is positioned horizontally. At the top of the chamber B, an opening is provided having a virus- and bacteria-tight hydrophobic filter 7, whereby pressure equalisation may be accomplished in the chamber B. Figure 3 shows a bag suspended in hooks underneath a measurement container 12 and provided with a discharge valve 13. It will appear that the height of the bag is limited, the foil sheets 9 lifting and fixating the lower edge of the bag. Likewise, the invention does not depend on the presence of NRV 6 or the ventilation opening 7, as the pressure equalisation may also take place through the inlet opening 4.

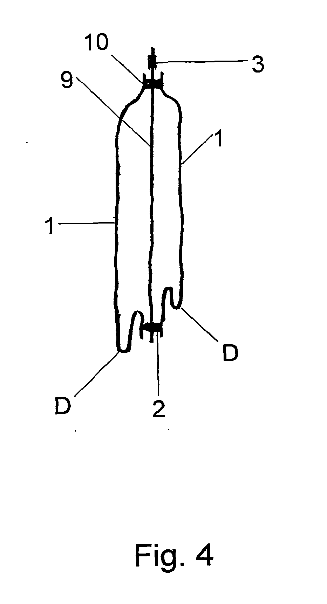

Figure 4 shows an embodiment of the bag seen from the side in the empty state, and it will appear how the bag A can be constructed on the basis of two separate foil sheets 1 that form the front of the bag (shown to the left in the drawing) and its rear, respectively, and a middle foil sheet 9 for carrying an essential part of the weight of the liquid. The foil sheets are welded along welding seam 2, 10, the foil sheet 9 being expanded between these two welding seams 2, 10 and the two remaining foil sheets 1 having a larger extent than the foil sheet 9 exhibiting folds along lines D. Upon filling of the bag, the foil sheets will bulge outwards and straighten out, the location of the bottom of the bag A being well-defined along the welding seam 2.

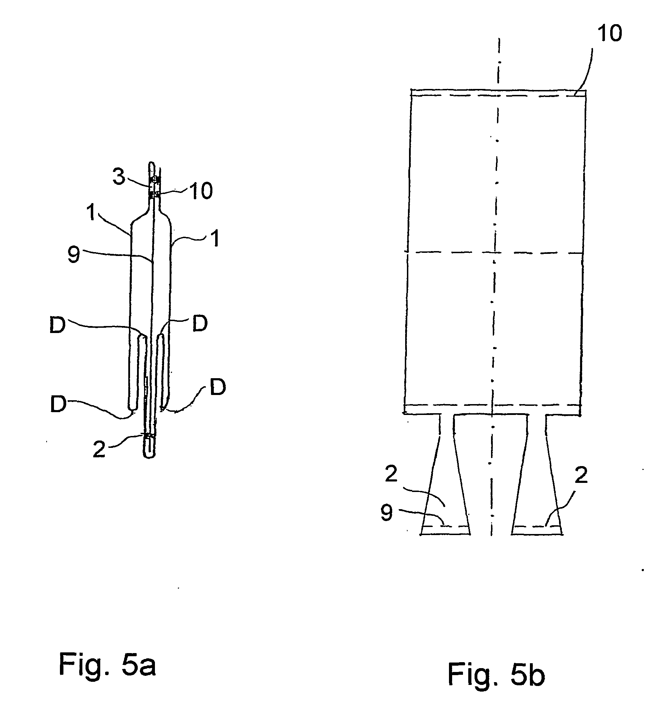

Figures 5a and 5b show an alternative embodiment, in which one coherent foil sheet is used, which is folded such that it is possible to establish a welding seam 2, 10 at the folds for mutually fixating the folded portions. At the one end the foil sheet can be cut as shown in Figure 5b for forming the means 9 that serve to carry the weight of the liquid.

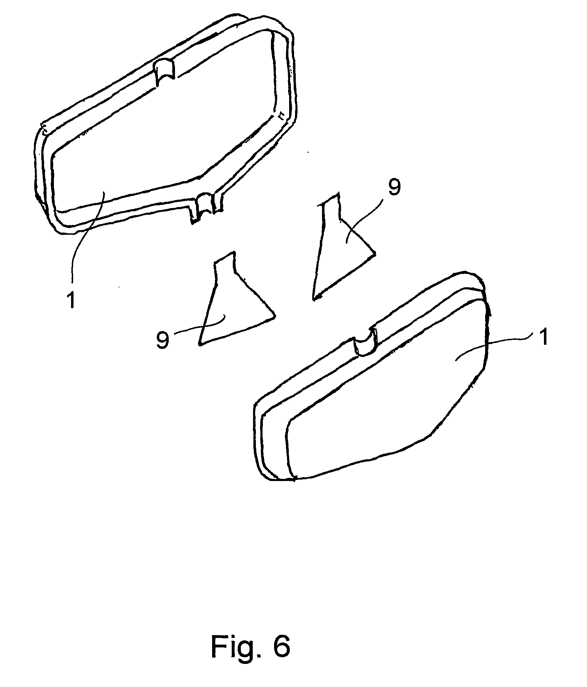

By the particular embodiment of the urine collecting bag shown in Figure 6 the foil elements are formed three-dimensionally, eg by thermoplastic shaping. Hereby a bag is formed that a) has a larger volume compared to the foil consumption; b) has a fixed geometry; and c) has a regular welding seam and hence a nicer appearance. Between the front and the rear means (9) are arranged that are configured for transferring a part of the weight of the contents of the bag to not shown holder devices at the top of the bag.

## Claims

1. A bag (A) for holding a liquid, in particular urine, said bag having an inlet opening (4) for the liquid and comprising a front (1) formed of a flexible material and a rear (1) formed of a flexible material and being connected to the front along an annularly extending connecting area (2, 10) for forming the sides of a chamber (B) for holding said liquid; and said bag (A) having at the top holder devices (3), by means of which the bag (A) can be carried in a suspended position, the front and rear of the bag (A) being configured for assuming a bulged shape when holding the liquid, **characterised in that**, interiorly of the chamber (B) between the flexible front and the flexible rear, means (9) are arranged for transferring at least a part of the weight of the liquid in the chamber (B) to the holder devices (3); and **in that** the front and/or the rear exhibit, in the empty state of the bag, at least one fold (D) that extends transversally of the bag (A).

2. A bag according to claim 1, **characterised in that** said means (9) are at least one flexible piece of material that extends between the connection (10) in the connecting area between the front and the rear most proximate the holder devices and the opposite connection between the front and the rear near the bottom of the bag.

3. A bag according to claim 2, **characterised in that** said piece of material (9) is a foil.

4. A bag according to claim 3, **characterised in that** the front (1), the rear (1) and said piece of material (9) consist of a coherent sheet of a foil.

5. A bag according to any one of claims 2-4, **characterised in that** the connection in the connecting area (2, 10) is established by welding;
that said flexible piece of material (9) for transferring at least a part of the weight of the liquid contained in the chamber (B) to the holder devices (3) is connected to the front (1) and the rear (1) along a part of the annularly extending connecting area at the welding; and
that the fold (D) is provided **in that** the distance between the welding (10) most proximate the holder devices (3) and an opposite welding most proximate the bottom of the bag (A) is smaller than the extent of the foils that form the front (1) and the rear (1), respectively, between these weldings.

6. A bag according to any one of the preceding claims, **characterised in that** the front (1) and the rear (1) consist of separate sheets of a foil.

7. A bag according to any one of the preceding claims, **characterised in that** the front (1) and the rear (1) consist of a coherent sheet of a foil.

8. A bag according to any one of the preceding claims, **characterised in that** the chamber (B) is elongate; and that said means (9) for transferring at least a part of the weight of the liquid contained in the chamber (B) to the holder devices (3) are arranged near the upright sides of the chamber (B).

9. A bag according to any one of the preceding claims 3-7, **characterised in that** the foils are manufactured from a plastics material.

10. A bag according to any one of the preceding claims 3-9, **characterised in that** the holder devices (3) are configured in the foil that forms the at least one flexible piece of material (9) for transferring at least a part of the weight of the liquid contained in the chamber (B).

11. A bag according to any one of the preceding claims, **characterised in that** the bag (A) has a separate outlet opening (8) for the liquid in the chamber (B).

12. A bag according to the preceding claim, **characterised in that** said means (9) for transferring at least a part of the weight of the liquid contained in the chamber (B) to the holder devices (3) are arranged along each of the upright sides of the chamber (B); and that the outlet openings (8) of the bag (A) for the liquid are arranged between said means (9).

13. A bag according to any one of the preceding claims, **characterised in that** the means (9) for transferring at least a part of the weight of the liquid contained in the chamber (B) for the holder devices (3) have a memory for shortened state.

14. A method of manufacturing a bag for holding a liquid, in particular urine, said bag having an inlet opening for the liquid and comprising a front formed of a flexible material and a rear formed of a flexible material and being connected to the front along an annularly extending connecting area for forming the sides of a chamber for holding said liquid; and said bag having, at the top, holder devices, by means of which the bag can be carried in a suspended position, the front and rear of the bag being configured for assuming a bulged shape when liquid has collected therein, **characterised in that**, in an area between the front and the rear, a separate foil (9) is inserted having a memory of a shortened state; that the connecting area (2, 10) is established by welding of the front (1), the rear (1) and the separate foil (9); and that the separate foil (9) is influenced to produce the shortened state, whereby the front (1) and the rear (1) are caused to exhibit at least one fold (D) that extends transversally of the bag (A).

## Patentansprüche

1. Beutel (A) zur Aufnahme einer Flüssigkeit, insbesondere Urin, wobei der Beutel eine Einlassöffnung (4) für die Flüssigkeit aufweist und eine aus einem flexiblen Material geformte Vorderseite (1) und eine Rückseite (1) umfasst, die aus einem flexiblen Material geformt ist und mit der Vorderseite entlang einem sich ringförmig erstreckenden Verbindungsbereich (2, 10) zur Bildung der Seiten einer Kammer (B) zur Aufnahme der Flüssigkeit verbunden ist; und wobei der Beutel (A) am oberen Teil Haltevorrichtungen (3) aufweist, mittels derer sich der Beutel (A) in hängender Lage tragen lässt, wobei die Vorderseite und die Rückseite des Beutels (A) so gestaltet sind, dass sie eine aufgewölbte Form annehmen, wenn sie die Flüssigkeit halten, **dadurch gekennzeichnet, dass** im Innern der Kammer (B), zwischen der flexiblen Vorderseite und der flexiblen Rückseite, Mittel (9) angeordnet sind, um zumindest einen Teil des Gewichts der Flüssigkeit in der Kammer (B) an die Haltevorrichtungen (3) zu übertragen; und **dass** die Vorderseite und/oder die Rückseite, bei Leerzustand des Beutels, mindestens eine Falte (D) aufweist bzw. aufweisen, die sich transversal zum Beutel (A) erstreckt.

2. Beutel nach Anspruch 1, **dadurch gekennzeichnet, dass** es sich bei den Mitteln (9) um mindestens ein flexibles Stück Material handelt, das sich zwischen der Verbindung (10) im Verbindungsbereich zwischen der Vorderseite und der Rückseite allernächst den Haltevorrichtungen und der gegenüberliegenden Verbindung zwischen der Vorderseite und der Rückseite nahe dem unteren Teil des Beutels erstreckt.

3. Beutel nach Anspruch 2, **dadurch gekennzeichnet, dass** das Stück Material (9) eine Folie ist.

4. Beutel nach Anspruch 3, **dadurch gekennzeichnet, dass** die Vorderseite (1), die Rückseite (1) und das Stück Material (9) aus einem kohärenten flächigen Teil einer Folie bestehen.

5. Beutel nach einem der Ansprüche 2 - 4, **dadurch gekennzeichnet, dass** die Verbindung im Verbindungsbereich (2, 10) durch Schweißen hergestellt wird;
**dass** das flexible Stück Material (9), das zumindest einen Teil des Gewichts der in der Kammer (B) enthaltenen Flüssigkeit an die Haltevorrichtungen (3) überträgt, mit der Vorderseite (1) und der Rückseite (1) entlang einem Teil des sich ringförmig erstreckenden Verbindungsbereichs beim Schweißen verbunden wird; und
**dass** die Falte (D) in der Form vorgesehen ist, dass die Entfernung zwischen der Schweißnaht (10) allernächst den Haltevorrichtungen (3) und einer gegenüberliegenden Schweißnaht allernächst dem unteren Teil des Beutels (A) kleiner ist als die Ausdehnung der Folien, welche jeweils die Vorderseite (1) und die Rückseite (1) bilden, zwischen diesen Schweißnähten.

6. Beutel nach einem der vorhergehenden Ansprüche, **dadurch gekennzeichnet, dass** die Vorderseite (1) und die Rückseite (1) aus separaten flächigen Teilen einer Folie bestehen.

7. Beutel nach einem der vorhergehenden Ansprüche, **dadurch gekennzeichnet, dass** die Vorderseite (1) und die Rückseite (1) aus einem kohärenten flächigen Teil einer Folie bestehen.

8. Beutel nach einem der vorhergehenden Ansprüche, **dadurch gekennzeichnet, dass** die Kammer (B) länglich ist; und **dass** die Mittel (9), die zumindest einen Teil des Gewichts der in der Kammer (B) enthaltenen Flüssigkeit an die Haltevorrichtungen (3) übertragen, nahe den aufrechten Seiten der Kammer (B) angeordnet sind.

9. Beutel nach einem der vorhergehenden Ansprüche 3 - 7, **dadurch gekennzeichnet, dass** die Folien aus einem Kunststoffmaterial hergestellt sind.

10. Beutel nach einem der vorhergehenden Ansprüche 3 - 9, **dadurch gekennzeichnet, dass** die Haltevorrichtungen (3) in der Folie ausgeführt sind, die das mindestes eine flexible Stück Material (9) bildet, das zumindest einen Teil des Gewichts der in der Kammer (B) enthaltenen Flüssigkeit überträgt.

11. Beutel nach einem der vorhergehenden Ansprüche, **dadurch gekennzeichnet, dass** der Beutel (A) eine separate Auslassöffnung (8) für die Flüssigkeit in der Kammer (B) hat.

12. Beutel nach dem vorhergehenden Anspruch, **dadurch gekennzeichnet, dass** die Mittel (9), die zumindest einen Teil des Gewichts der in der Kammer (B) enthaltenen Flüssigkeit an die Haltevorrichtungen (3) übertragen, entlang jeder der aufrechten Seiten der Kammer (B) angeordnet sind; und **dass** die Auslassöffnungen (8) des Beutels (A) für die Flüssigkeit zwischen den Mitteln (9) angeordnet sind.

13. Beutel nach einem der vorhergehenden Ansprüche, **dadurch gekennzeichnet, dass** die Mittel (9), die zumindest einen Teil des Gewichts der in der Kammer (B) enthaltenen Flüssigkeit an die Haltevorrichtungen (3) übertragen, ein Gedächtnis für einen verkleinerten Zustand haben.

14. Verfahren zur Herstellung eines Beutels zur Aufnahme einer Flüssigkeit, insbesondere Urin, wobei der Beutel eine Einlassöffnung für die Flüssigkeit aufweist und eine aus einem flexiblen Material geformte Vorderseite und eine Rückseite umfasst, die aus einem flexiblen Material geformt ist und mit der Vorderseite entlang einem sich ringförmig erstreckenden Verbindungsbereich zur Bildung der Seiten einer Kammer zur Aufnahme der Flüssigkeit verbunden ist; und wobei der Beutel, am oberen Teil, Haltevorrichtungen aufweist, mittels derer sich der Beutel in hängender Lage tragen lässt, wobei die Vorderseite und die Rückseite des Beutels so gestaltet sind, dass sie eine aufgewölbte Form annehmen, wenn sich Flüssigkeit in demselben gesammelt hat, **dadurch gekennzeichnet, dass,** in einen Bereich zwischen der Vorderseite und der Rückseite, eine separate Folie (9) eingefügt wird, die ein Gedächtnis für einen verkleinerten Zustand besitzt; **dass** der Verbindungsbereich (2, 10) durch Verschweißen der Vorderseite (1), der Rückseite (1) und der separaten Folie (9) hergestellt wird; und dass die separate Folie (9) beeinflusst wird, damit sie den verkleinerten Zustand herstellt, wodurch die Vorderseite (1) und die Rückseite (1) veranlasst werden, mindestens eine Falte (D) aufzuweisen, die sich transversal zum Beutel (A) erstreckt.

## Revendications

1. Sachet (A) pour retenir un liquide, en particulier l'urine, ledit sachet ayant une ouverture d'entrée (4) pour le liquide et comprenant un avant (1) réalisé en un matériau flexible et un arrière (1) réalisé en un matériau flexible et étant relié à l'avant le long d'une zone de connexion s'étendant annulairement (2, 10) pour former les côtés d'une chambre (B) pour retenir ledit liquide ; et ledit sachet (A) ayant sur le dessus des dispositifs de support (3) au moyen desquels le sachet (A) peut être porté dans une position suspendue, l'avant et l'arrière du sachet (A) étant configuré pour avoir une forme bombée lors de la retenue du liquide, **caractérisé en ce que**, à l'intérieur de la chambre (B) entre l'avant flexible et l'arrière flexible, des moyens (9) sont agencés pour transférer au moins une partie du poids du liquide dans la chambre (B) aux dispositifs de support (3) ; et **en ce que** l'avant et/ou l'arrière présente, à l'état vide du sachet, au moins un pli (D) qui s'étend transversalement du sachet (A).

2. Sachet selon la revendication 1, **caractérisé en ce que** lesdits moyens (9) sont au moins une pièce de matériau flexible qui s'étend entre la connexion (10) dans la zone de connexion entre l'avant et l'arrière à la plus grande proximité des dispositifs de support et la connexion opposée entre l'avant et l'arrière près du fond du sachet.

3. Sachet selon la revendication 2, **caractérisé en ce que** ladite pièce de matériau (9) est une feuille.

4. Sachet selon la revendication 3, **caractérisé en ce que** l'avant (1), l'arrière (1) et ladite pièce de matériau (9) sont formés par une feuille cohérente.

5. Sachet selon l'une des revendications 2 à 4, **caractérisé en ce que** la connexion dans la zone de connexion (2, 10) est établie par soudage ; **en ce que** ladite pièce de matériau flexible (9) pour transférer au moins une partie du poids du liquide se trouvant dans la chambre (B) aux dispositifs de support (3) est reliée à l'avant (1) et à l'arrière (1) le long d'une partie de la zone de connexion s'étendant annulairement à la soudure; et
**en ce que** le pli (D) est réalisé **en ce que** la distance entre la soudure (10) la plus proche des dispositifs de support (3) et une soudure opposée la plus proche du fond du sachet (A) est plus petite que l'étendue des feuilles qui forment l'avant (1) et l'arrière (1), respectivement, entre ces soudures.

6. Sachet selon l'une des revendications précédentes, **caractérisé en ce que** l'avant (1) et l'arrière (1) sont réalisés par des feuilles séparées.

7. Sachet selon l'une des revendications précédentes, **caractérisé en ce que** l'avant (1) et l'arrière (1) sont réalisés en une feuille cohérente.

8. Sachet selon l'une des revendications précédentes, **caractérisé en ce que** la chambre (B) est oblongue ; et **en ce que** lesdits moyens (9) pour transférer au moins une partie du poids du liquide se trouvant dans la chambre (B) aux dispositifs de support (3) sont agencés près des côtés verticaux de la chambre (B).

9. Sachet selon l'une des revendications précédentes 3 à 7, **caractérisé en ce que** les feuilles sont fabriquées en matériau plastique.

10. Sachet selon l'une des revendications précédentes 3 à 9, **caractérisé en ce que** les dispositifs de support (3) sont configurés dans la feuille qui forme au moins une pièce de matériau flexible (9) pour transférer au moins une partie du poids du liquide se trouvant dans la chambre (B).

11. Sachet selon l'une des revendications précédentes, **caractérisé en ce que** le sachet (A) présente une ouverture de sortie séparée (8) pour le liquide dans la chambre (B).

12. Sachet selon la revendication précédente, **caractérisé en ce que** lesdits moyens (9) pour transférer au moins une partie du poids du liquide se trouvant dans la chambre (B) aux dispositifs de support (3) sont agencés le long de chacun des côtés verticaux de la chambre (B) ; et **en ce que** les ouvertures de sortie (8) du sachet (A) pour le liquide sont agencées entre lesdits moyens (9).

13. Sachet selon l'une des revendications précédentes, **caractérisé en ce que** les moyens (9) pour transférer au moins une partie du poids du liquide se trouvant dans la chambre (B) pour les dispositifs de support (3) ont une mémoire pour un état raccourci.

14. Procédé de fabrication d'un sachet pour retenir un liquide, en particulier l'urine, ledit sachet ayant une ouverture d'entrée pour le liquide et comprenant un avant réalisé en un matériau flexible et un arrière réalisé en un matériau flexible et étant relié à l'avant le long d'une zone de connexion s'étendant annulairement pour former les côtés d'une chambre pour retenir ledit liquide ; et ledit sachet ayant, sur le dessus, des dispositifs de support au moyen desquels le sachet peut être porté en position suspendue, l'avant et l'arrière du sachet étant configurés pour avoir une forme bombée lorsque le liquide a été recueilli dans celui-ci, **caractérisé en ce que**, dans une zone entre l'avant et l'arrière, une feuille séparée (9) est insérée ayant une mémoire d'un état raccourci ; **en ce que** la zone de connexion (2, 10) est établie par le soudage de l'avant (1), de l'arrière (1) et de la feuille séparée (9) ; et **en ce que** la feuille séparée (9) est influencée pour produire l'état raccourci, par quoi l'avant (1) et l'arrière (1) sont amenés à présenter au moins un pli (D) qui s'étend transversalement du sachet (A).
